# EUROPEAN PATENT SPECIFICATION

(11) **EP 4 525 904 B1**
(45) Date of publication and mention of the grant of the patent: **06.05.2026**
(21) Application number: 23738110.8
(22) Date of filing: 17.05.2023
(51) Int. Cl.: A61K 38/00, A61K 38/04, A61K 38/10, A61P 19/02, C07K 7/08, C07K 7/64

(54) **ENGINEERED SYNTHETIC PEPTIDES, COMPOSITIONS COMPRISING SAME, AND METHODS OF USE THEREOF FOR TREATING MALIGNANT GLIOMAS**
MANIPULIERTE SYNTHETISCHE PEPTIDE, ZUSAMMENSETZUNGEN DAMIT UND VERFAHREN ZUR VERWENDUNG DAVON ZUR BEHANDLUNG VON MALIGNEN GLIOMEN
PEPTIDES SYNTHÉTIQUES MODIFIÉS, COMPOSITIONS LES COMPRENANT, ET LEURS PROCÉDÉS D'UTILISATION POUR TRAITER DES GLIOMES MALINS

(30) Priority: 19.05.2022 PL 44122922
(43) Date of publication of application: 26.03.2025
(73) Proprietor: Instytut Biologii Doswiadczalnej Im. Marcelego Nenckiego Polska Akademia Nauk, 02-093 Warszawa (PL)
(72) Inventor: POLESZAK, Katarzyna, 01-157 Warszawa (PL); PASIERBINSKA, Maria, 05-800 Pruszkow (PL); ELLERT-MIKLASZEWSKA, Aleksandra, 05-806 Komorow (PL); PILANC-KUDLEK, Paulina, 02-237 Warszawa (PL); WISNIEWSKI, Pawel, 05-082 Blizne Jasinskiego (PL); KAMINSKA-KACZMAREK, Bozena, 05-806 Komorow (PL)
(74) Representative: Patpol Kancelaria Patentowa Sp. z o.o.
(86) International application number: PCT/IB2023/055089
(87) International publication number: WO 2023/223241

(56) References cited:
- EP-A1- 3 886 888
- WO-A2-2013/072901
- US-A1- 2013 309 193

## Description

### Field of the invention

The invention relates to engineered synthetic peptides, compositions comprising same, and methods of use thereof in the treatment of macrophage-infiltrated tumours, such as malignant gliomas.

### Prior art

Gliomas are the most common tumours of the central nervous system (CNS) in adults. They account for 40% of all cancers and 78% of malignant tumours of the central nervous system. According to the classification made by the World Health Organization, gliomas are divided into 4 groups based on the degree of malignancy determined on the basis of various histological parameters and genetic changes in cancer cells. Stage IV of glioblastoma multiforme (GBM) is the most common glioma in adults and accounts for 69% of all cases of these tumours. Due to late diagnosis and lack of effective therapy, GBM regrows shortly after diagnosis and is incurable. The treatment recommended by the WHO and the National Health Fund includes the maximum acceptable surgical removal of the tumour, radiotherapy and chemotherapy, but due to the ineffective removal of the diffuse tumour and the resistance of cancer cells to treatment, the tumours recur after 6 months and are usually even more malignant. The average survival of patients is 14 months (Furnari et al., 2007).

Many clinical trials are conducted on potential substances for the treatment of glioblastoma multiforme. So far, none of them has shown the expected effectiveness in extending or improving the patients' life. Even immunotherapy using checkpoint inhibitors, which have proven effective in the treatment of other cancers such as melanoma and lung cancer, has not brought benefits to patients in the treatment of glioma. This is related to the immunosuppressive microenvironment of glioma, referred to as *"cold tumour microenvironment"* characterized by low numbers of incoming cytotoxic T cells. In malignant gliomas, local and systemic immunosuppression is generated with myeloid cells recruited into the tumour. The glioblastoma-promoting phenotype of these cells is characterized by activation of pathways controlling microglial motility and phagocytosis, lack of inflammatory and anticancer responses, which promotes the influx of immunosuppressive myeloid cells (e.g. MDSC) and regulatory T cells, and blocks the activity of cytotoxic T or NK cells. Studies of the gene expression profile in microglia/macrophages in the tumour and in peripheral blood monocytes suggest that these cells acquire immunosuppressive and pro-invasive properties only in the presence of glioma (Gabrusiewicz et al., 2016; Sielska et al., 2021). Pharmacological (Sliwa et al., 2007; Gabrusiewicz et al., 2011, Markovic et al., 2011) or genetic (Zhai et al., 2011) elimination of microglia/macrophages in experimental gliomas significantly reduces tumour growth, indicating an important role of these cells in the pathogenesis of gliomas. Macrophage/microglia number in human gliomas correlate with the degree of malignancy of the tumour and shorter survival.

The inventors of the present invention were among the first to show that glioblastoma cells attract myeloid cells, mainly microglia and peripheral monocytes/macrophages, to the tumour and transform them into cells that are immunosuppressive and support tumour invasiveness and progression (Sliwa et al. 2007, Wesolowska et al. 2008, Markovic et al. 2009; Gabrusiewicz et al. 2011, 2015; Ochocka, Segit et al. 2021). These cells referred to as GAMs (*glioma-associated microglia*/*macrophages*) often constitute up to 30% of the tumour mass. The inventors of the present invention have demonstrated that glioblastoma cells secrete proteins that reprogramme myeloid cells into cells that promote invasiveness, angiogenesis, and block the antitumour response. One of the key factors forming the pro-invasive GAM phenotype is osteopontin (SPP1, OPN) (Ellert-Miklaszewska et al., 2016). Silencing of *Spp1* (a gene encoding osteopontin) expression in glioma cells inhibited the growth of glioma in rats and human glioma cells in an animal model using immunodeficient mice Crl:NU(Ico)-*Foxn1^{nu}*. This was accompanied by a decrease in the number of amoeboid GAMs with a pro-cancer phenotype (Gieryng et al. unpublished). Increased *SPP1* expression correlates with the degree of malignancy of the tumour, being the highest in GBM (Kijewska et al. 2017), and the concentration of protein in the tumour tissue and serum of patients with glioblastoma is a negative prognostic factor (Atai et al., 2011; Kijewska et al. 2017). Osteopontin stimulates the proliferation and invasiveness of cancer cells, as well as the process of angiogenesis, mediated by the alphavbeta3 and alphavbeta5 integrin receptors.

The inventors of the present invention have previously shown that the short RGD peptide blocks the interaction between rat glioma and microglia in co-culture by blocking reprogramming towards pro-invasive microglia (Ellert-Miklaszewska et al., 2016). A new therapeutic strategy has been proposed based on the use of short peptides that disrupt the interaction between OPN and its receptors, and thus block signalling pathways crucial for the invasiveness and progression of glioblastoma. A series of synthetic peptides was designed and tested, and peptides that selectively bind to the OPN protein, block its binding to the appropriate receptors on microglia, and block the activation of these receptors and downstream signalling pathways, resulting in inhibition of glioblastoma invasiveness and growth were identified. By administering such a short peptide inhibitor directly into a brain tumour, these compounds will not have to cross the blood-brain barrier. In addition, intratumoural administration will minimize any non-specific toxicity. Moreover, it is also possible to encapsulate these short peptides in carriers that will stabilize and enable them to cross the blood-brain barrier when administered systemically. Therefore, therapy using targeted, short peptides has a good chance of replacing or enriching ineffective methods of treating patients with malignant glioma.

The inventors of the present invention have demonstrated that the OPN is a key factor forming the pro-invasive GAM phenotype in gliomas. In view of the failures so far in finding effective therapeutic agents to combat malignant gliomas, blocking key factors such as OPN, which control the accumulation of microglia and migration of macrophages and other suppressive cells of the immune system attracted from the blood or bone marrow into the tumour microenvironment, is a new promising therapeutic strategy. In this context, short, interfering peptides, due to their high specificity and effectiveness in blocking glioma-microglia interactions, may be the answer to the unmet medical need, which is the effective therapy of glioma and other cancers characterized by an immunosuppressive microenvironment.

In addition, various therapeutic substances are currently being sought that could increase the effectiveness of immunotherapy, which is not effective in the treatment of malignant gliomas. The inventors of the present invention obtained results showing that in a mouse model, the RGD peptide (with a similar mechanism of action) administered intratumorally cooperated with the blockade of PD1. The combination of RGD+anti-PD1 antibody inhibited glioblastoma growth (Pilanc et al., unpublished). The peptides proposed in the present invention are an ideal candidate due to their ability to modify the immunosuppressive tumour microenvironment and restore the antitumour response. The use of an OPN blocking peptide would effectively inhibit invasiveness in gliomas and increase the effectiveness of immunotherapy with an antibody directed against immune checkpoints (e.g. anti-PD1), which should enable the infiltration and proper functioning of cytotoxic T lymphocytes and thus restore the endogenous antitumour response.

The binding of adhesion molecules, chemokines, growth factors or extracellular matrix proteins to specific receptors creates a communication network between the tumour and the microenvironment. The most specific way to block this type of protein-protein interactions is the use of short peptides (Ellert-Miklaszewska et al., 2017). One of the attractive therapeutic targets in cancer are integrins, membrane receptors that become activated after binding to a ligand and transmit signals inside the cell through FAK, AKT and ERK kinases that regulate migration, invasiveness, proliferation and cell viability (Danen, 2013). Research conducted by the inventors of the present invention has shown that integrins play a role in the glioma-induced reprogramming of microglia into cells that support the development of gliomas and enable the function of extracellular osteopontin. The peptide designed by the inventors of the present invention (competitive inhibitor of integrins, containing the RGD motif) blocked the rat glioma-microglia interaction in co-culture *in vitro* (Ellert-Miklaszewska et al., 2016) and in a mouse glioblastoma model (unpublished data). Another protein secreted by glioblastoma in a mouse model of this tumour, Periostin, induced the influx of myeloid cells into the tumour via the integrin alphavbeta3. Inhibition of periostin by a RGD-containing peptide resulted in tumour limitation (Zhou et al., 2015). Interestingly, in models of tumours developing outside the central nervous system, mice lacking the gene encoding the integrin beta3 (Itgb3-/-) showed decreased macrophage infiltration accompanied by tumour growth (Taverna et al., 2004). The best-known integrin inhibitor is the cyclic pentapeptide containing the RGD motif - cilengitide (EMD 121974, cyclo-(Arg-Gly-Asp-DPhe-NMe-Val), MerckKGaA, Darmstadt, Germany), which was the first inhibitor of alphav integrins has been entered into phase III clinical trials (Weller et al. 2016). Studies of the use of cilengitide in a large number of patients, however, did not show the effectiveness of this substance, mainly due to the lack of stratification of patients according to the expression of integrins.

The presented strategy for the treatment of malignant gliomas is based on the unique scientific results obtained by the inventors of the present invention showing that the modification of microglial and macrophage functions in the extracellular environment of the tumour can be an effective therapy against gliomas. The peptides of this invention are unique synthetic molecules and have been designed and screened for their anti-glioma properties. The approach using the peptides according to the invention is innovative on a global scale, and its results may be important not only for revolutionizing the treatment of glioblastomas, but may also potentially contribute to progress in the treatment of other cancers.

In order to assess the innovativeness of glioblastoma therapy using the peptides according to the invention, the inventors used the scale of pharmaceutical innovations proposed by Steven Seget. The following categories of innovation among new drugs can be distinguished:
- **new market innovations** - new drugs that meet a previously unmet market (clinical) need
- **innovations redefining the category** - drugs that significantly change the way a given disease is managed
- **second generation innovations** - improved versions of known drugs, having a clear advantage over the originals
- **incremental innovations** - improved versions of known drugs, with a slight advantage in effectiveness or slightly lower side effects than the originals (also known as "me-too drugs")
- **formulation innovations -** innovations that do not introduce therapeutic effects, but facilitate dosing and taking drugs

All pharmacological therapies of gliomas that are currently used or may soon be used in clinical practice fall under the definition of "category redefining innovations" or even "incremental innovations" (registration of malignant glioma as the next consecutive indication for known drugs or the use of slightly improved approaches compared to the standards in the treatment of other cancers). Meanwhile, the use of the peptides according to the invention certainly fits the definition of "new market innovation", because it targets the mechanisms underlying the process of cancer development, including the escape from the control of the immune system, and thus offers a radically new approach to the treatment of this disease.

Although osteopontin inhibition has been shown to be effective in various models of preclinical diseases, such as rheumatoid arthritis, lung cancer, non-alcoholic steatohepatitis, and many research groups and pharmaceutical companies have been working on the development of OPN binding antibodies, so far, such antibodies are not used in clinical practice. Astelas Pharma has been developing the ASK8007 antibody as a treatment for rheumatoid arthritis, however, clinical trials have not demonstrated the effectiveness of this substance in patients (Farrokhi et al., 2018). It was observed that the OPN protein persisted longer in the body, which was associated with an increased resistance of the OPN-ASK8007 antibody complex to enzymatic digestion, an increase in the half-life of the OPN protein, leading to an increase in its concentration. In turn, Pfizer studied the use of the AOM1 antibody in a model of lung cancer metastasis (Shojaei et al., 2012; Farrokhi et al., 2018). Farrokhi et al. showed that the serum level of OPN is very high and the degradation time of this protein is very fast. Therefore, antibodies directed against OPN would need to have increased pharmacokinetics than traditional antibodies, and would also need to be administered repeatedly at higher doses. The antibodies are immunogenic and frequent administration causes side effects, in addition, the use of higher doses could potentially enhance the processes against which they are administered. These are potential reasons why anti-OPN antibodies are not used in the clinical practice. The advantage of short peptides over antibodies is that they are safer, more specific, and can be administered at higher doses. The peptides are cheaper to produce, which is an important consideration when higher doses and more frequent administrations are required.

### Summary of the invention

The present invention discloses particular peptides, compositions comprising same, and methods of use thereof to treat a subject with cancer that is heavily infiltrated by macrophages with tumour-promoting activity that contribute to tumour growth or maintenance. Thus, the present invention provides a means for treating tumours with macrophages having tumour-promoting activity, such as malignant gliomas.

In accordance with one aspect of the invention, particular peptides that block OPN activity are disclosed, wherein said peptides comprise an amino acid sequence selected from the group consisting of: the amino acid sequence set forth in SEQ ID NO: 1 (YDGKNMSSLYNFTG), the amino acid sequence set forth in SEQ ID NO: 2 (SLYNFTGEQMAAYF); and the amino acid sequence set forth in SEQ ID NO: 3 (SKYDPNVYSIKYNN). Each possibility is a separate embodiment in which said peptide consists of 14 amino acids. According to an embodiment, the peptide studied in detail consists of 14 amino acids.

In yet another embodiment, the synthetic peptide consists of the sequence set forth in SEQ ID NO: 1 or an analogue or derivative thereof. Alternatively, the synthetic peptide consists of the sequence set forth in SEQ ID NO: 2 or an analogue or derivative thereof. Alternatively, the synthetic peptide consists of the sequence set forth in SEQ ID NO: 3 or an analogue or derivative thereof.

In yet another embodiment, the peptide is a cyclic peptide.

According to yet another embodiment, the peptide may be linked to another peptide or carrier to stabilize the peptide for systemic administration.

In yet another embodiment, the peptide may be linked to another peptide or carrier capable of crossing the blood-brain barrier when administered systemically.

In accordance with yet another aspect of the invention, there is provided a pharmaceutical composition comprising a synthetic peptide selected from the group consisting of: an amino acid sequence set forth in SEQ ID NO: 1, the amino acid sequence set forth in SEQ ID NO: 2 and the amino acid sequence set forth in SEQ ID NO: 3, substantially as disclosed in the preceding aspect of the invention, and a pharmaceutically acceptable carrier.

According to one embodiment, the pharmaceutically acceptable carrier is selected from the group consisting of: an aqueous solution, vegetable oil, alcohol, polyethylene glycol, propylene glycol or glycerine. Each possibility is a separate embodiment.

According to another embodiment, the pharmaceutical composition is for use in the treatment of glioma.

In yet another embodiment, the glioblastoma is selected from the group consisting of: ependymoma, astrocytoma, oligodendroglioma, glioblastoma multiforme, or mixed glioblastoma. Each possibility is a separate embodiment.

According to yet another aspect of the invention, any of the above peptides or a mixture thereof for use in the treatment of glioblastoma is provided.

In yet another embodiment, the glioblastoma is selected from the group consisting of: ependymoma, astrocytoma, oligodendroglioma, glioblastoma multiforme, or mixed glioblastoma. Each possibility is a separate embodiment. According to yet another embodiment, the treatment of glioblastoma is selected from the group consisting of: reducing phagocytosis, reducing motility, reducing the influx and proliferation of tumour-infiltrating macrophages with protumorigenic activity, and reducing the secretion of pro-inflammatory cytokines or chemokines by said macrophages. Each possibility is a separate embodiment.

According to another aspect of the invention, there is provided a kit for use in the treatment of glioblastoma comprising a pharmaceutical composition as discussed above or a synthetic peptide as illustrated above.

Other objects, features and advantages of the present invention will become apparent from the following description.

### Description of the figures

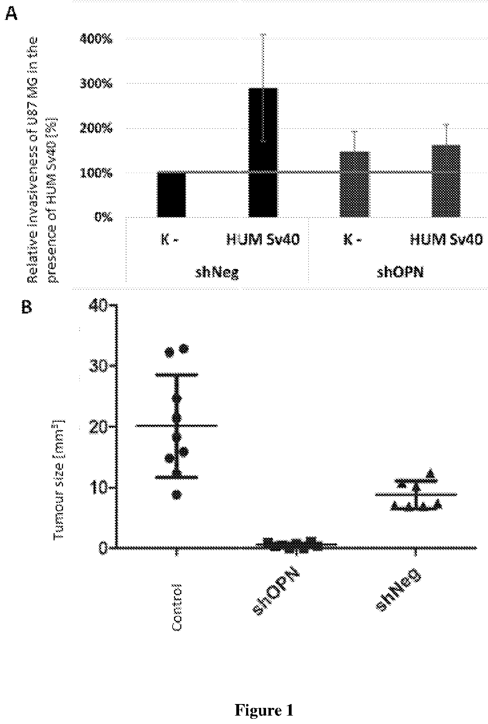

**Figure 1****. Effect of silencing OPN expression in U87 MG RFP glioma cells on cell invasiveness *in vitro* and tumour growth *in vivo.*** (A) Evaluation of the invasiveness of U87 shNeg and shOPN glioma cells stimulated by SV40 human microglial cells. (B) Tumour sizes after implantation of U87 MG RFP (control), shOPN and shNeg cells into Athymic Nude mice.

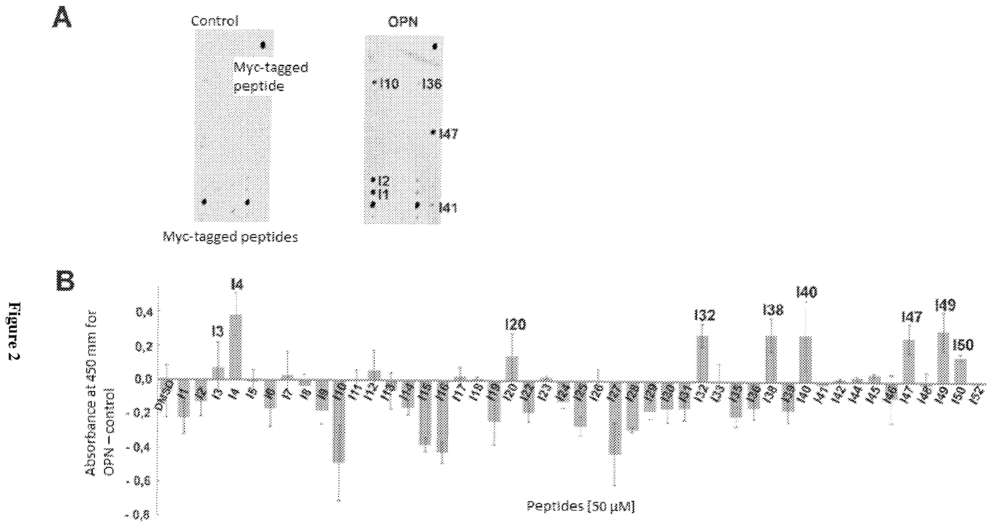

**Figure 2****. OPN-binding peptides. (A)** OPN binding peptides were identified using peptide microarrays comprising synthetic peptides. Images of peptide microarrays after incubation with PBS buffer (control), with rhOPN and after immunochemical staining. Control peptides (peptide containing Myc tag - positive control) and peptides with the highest signal were marked on the microarray image. **(B)** OPN binding peptides identified using ELISA. Peptides with the strongest binding to OPN were selected. Means ± SD are shown, standard deviations were calculated from three independents measurements.

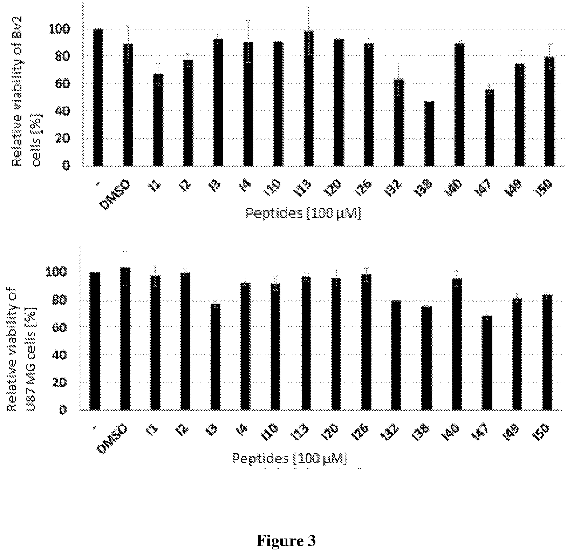

**Figure 3****. Effect of OPN binding peptides on viability of BV2 microglial cell and U87 MG glioma.** Cell viability was tested using the MTT metabolism assay. U87 MG and BV2 cells were incubated with the peptides at a concentration of 100 µM for 18 h. Peptides showing no cytotoxicity were selected.

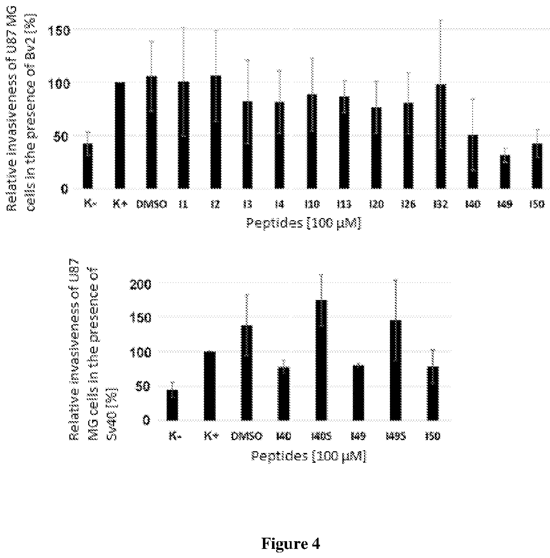

**Figure 4****. Effect of OPN binding peptides on U87 MG cell invasiveness in the presence of murine BV2 and human SV40 microglial cells.** (K-) - negative control - invasiveness of U87 MG cells without microglia; (K+) - positive control, invasiveness of U87 MG dependent on BV2 or SV40 microglia, defined as 100%; DMSO - cells treated with 0.2% DMSO - peptide solvent. Results are presented as means ± standard deviation calculated from three independent biological replicates. Selected peptides block microglia-dependent invasiveness.

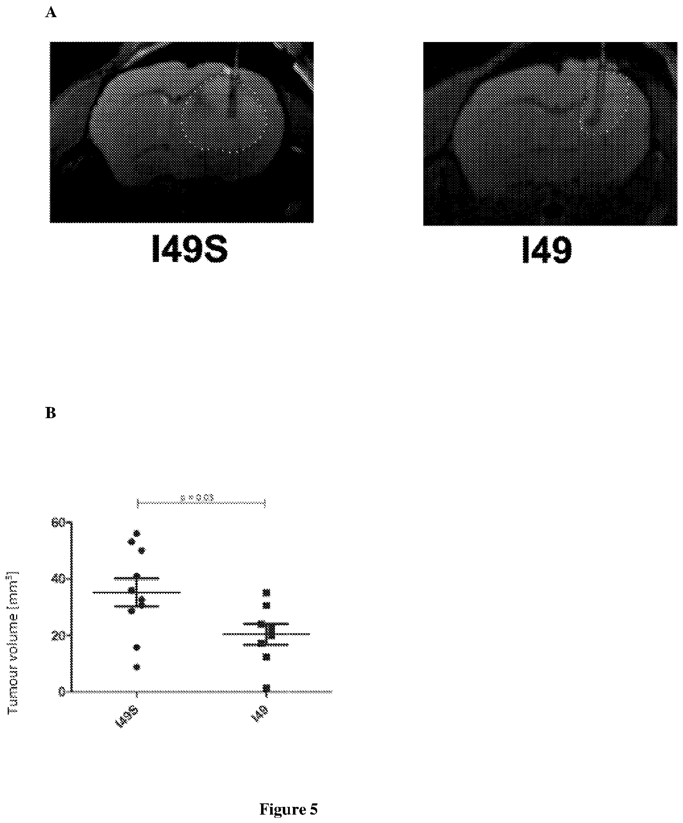

**Figure 5****. Effect of peptide 149 on glioblastoma growth in mice. (A)** Representative images obtained after vital MRI imaging. The pictures show a section through the center of the tumour, showing the cannula supplying the peptide from the osmotic pump. **(B)** Tumour volume (n=10) calculated using OsiriX DICOM Viewer from MRI scans.

### Detailed description of the invention

The present invention discloses particular peptides and pharmaceutical compositions which may be used in treating a subject having a cancer that is infiltrated by macrophages ("infiltrating macrophages") that exhibit protumorigenic activity. Infiltrating macrophages having pro-tumoral activity may participate in matrix remodelling, invasion, angiogenesis and suppression of adaptive immunity and may proliferate, be phagocytic and motile. Infiltrating macrophages with pro-tumoral activity and those that may contribute to tumour growth, maintenance or metastasis are present in tumours such as malignant tumours, e.g. brain tumours such as gliomas.

The invention is based on the unexpected findings that glioblastoma cells secrete proteins that reprogram myeloid cells into cells that promote invasiveness, angiogenesis, and block the antitumour response. One of the key factors forming the pro-invasive GAM phenotype is osteopontin, also known as SPP1, OPN, BNSP; BSPI or ETA-1.

The amino acid sequence of the human osteopontin precursor is provided under GenBank accession number: AAH17387.1 (SEQ ID NO: 4; mriavicfcl lgitcaipvk qadsgsseek qlynkypdav atwlnpdpsq kqnllapqna vsseetndfk qetlpsksne shdhmddmdd eddddhvdsq dsidsndsdd vddtddshqs deshhsdesd elvtdfptdl patevftpvv ptvdtydgrg dsvvyglrsk skkfrrpdiq ypdatdedit shmeseelng aykaipvaqd lnapsdwdsr gkdsyetsql ddqsaethsh kqsrlykrka ndesnehsdv idsqelskvs refhshefhs hedmlvvdpk skeedkhlkf risheldsas sevn) and is encoded by a nucleotide sequence provided with a GenBank accession number: BC017387.1 (SEQ ID NO: 5; ggggagcagc aggaggaggc agagcacagc atcgtcggga ccagactcgt ctcaggccag ttgcagcctt ctcagccaaa cgccgaccaa ggaaaactca ctaccatgag aattgcagtg atttgctttt gcctcctagg catcacctgt gccataccag ttaaacaggc tgattctgga agttctgagg aaaagcagct ttacaacaaa tacccagatg ctgtggccac atggctaaac cctgacccat ctcagaagca gaatctccta gccccacaga atgctgtgtc ctctgaagaa accaatgact ttaaacaaga gacccttcca agtaagtcca acgaaagcca tgaccacatg gatgatatgg atgatgaaga tgatgatgac catgtggaca gccaggactc cattgactcg aacgactctg atgatgtaga tgacactgat gattctcacc agtctgatga gtctcaccat tctgatgaat ctgatgaact ggtcactgat tttcccacgg acctgccagc aaccgaagtt ttcactccag ttgtccccac agtagacaca tatgatggcc gaggtgatag tgtggtttat ggactgaggt caaaatctaa gaagtttcgc agacctgaca tccagtaccc tgatgctaca gacgaggaca tcacctcaca catggaaagc gaggagttga atggtgcata caaggccatc cccgttgccc aggacctgaa cgcgccttct gattgggaca gccgtgggaa ggacagttat gaaacgagtc agctggatga ccagagtgct gaaacccaca gccacaagca gtccagatta tataagcgga aagccaatga tgagagcaat gagcattccg atgtgattga tagtcaggaa ctttccaaag tcagccgtga attccacagc catgaatttc acagccatga agatatgctg gttgtagacc ccaaaagtaa ggaagaagat aaacacctga aatttcgtat ttctcatgaa ttagatagtg catcttctga ggtcaattaa aaggagaaaa aatacaattt ctcactttgc atttagtcaa aagaaaaaat gctttatagc aaaatgaaag agaacatgaa atgcttcttt ctcagtttat tggttgaatg tgtatctatt tgagtctgga aataactaat gtgtttgata attagtttag tttgtggett catggaaact ccctgtaaac taaaagcttc agggttatgt ctatgttcat tctatagaag aaatgcaaac tatcactgta ttttaatatt tgttattctc tcatgaatag aaatttatgt agaagcaaac aaaatacttt tacccactta aaaagagaat ataacatttt atgtcactat aatcttttgt tttttaagtt agtgtatatt ttgttgtgat tatctttttg tggtgtgaat aaatctttta tcaaaaaaaa aaaaaaaaaa aaaaaaaaa). Osteopontin is an extracellular structural protein expressed in bone as well as in other tissues.

As used herein, the term "OPN inhibitor" refers to a substance that blocks at least one biological activity of OPN. According to some embodiments, the OPN inhibitor is a substance that blocks the progression of a cancer, e.g., glioblastoma, for example by slowing down the progression of the cancer by at least 10%, 20%, 30%, 40%, 50%, 60%, 70%, 80% 90%, 95%, 99% or 100%, relative to tumour progression in the absence of an OPN inhibitor. The OPN inhibitor may also be an inhibitor that stabilizes tumour (e.g., glioblastoma) size or reduces it by at least 10%, 20%, 30%, 40%, 50%, 60%, 70%, 80%, 90%, 95 %, 99% 100% (2 fold), 3 fold, 5 fold or more. An OPN inhibitor may, for example, reduce tumour infiltration by macrophages or microglia; reduce the stimulation and/or reprogramming of tumour-infiltrating macrophages into cells exhibiting protumorigenic activity; and/or reduce angiogenesis in a tumour. An OPN inhibitor may have one of the following characteristics: (i) blocking the production or synthesis of OPN, e.g., by tumour cells; (ii) neutralizing OPN activity; (iii) preventing or blocking the binding of OPN to its receptor; (iv) preventing or blocking the interaction of OPN with cell membrane surface proteins, (v) blocking the signal transduction pathway that is activated by the binding of OPN to its receptor on macrophages or microglia, or (vi) blocking production or synthesis of the OPN receptor, e.g., macrophages or microglia. The OPN inhibitor may be protein- or peptide based. The OPN inhibitor may also be a substance that blocks OPN expression, e.g. a blocking nucleic acid, e.g., siRNA, shRNA, antisense molecule, ribozyme or aptamer. As used herein, the term "substance" refers to any type of molecule or complex of molecules, such as macromolecules or small molecules.

An OPN inhibitor may block the biological activity of OPN by at least 10%, 20%, 30%, 40%, 50%, 60%, 70%, 80%, 90%, 95%, 99% or 100%. For example, an OPN inhibitor may reduce the interaction between an OPN and its receptor by a factor of at least 10%, 20%, 30%, 40%, 50%, 60%, 70%, 80%, 90%, 95%, 99% or 100%. An OPN inhibitor may also be a substance that blocks the expression of the OPN protein or proteins with which OPN interacts (e.g. alphavbeta3 and alphavbeta5 integrin receptors) and may e.g., reduce its expression by a factor of at least 10%, 20%, 30%, 40%, 50% , 60%, 70%, 80%, 90%, 95%, 99% or 100%.

### Blocking peptides or proteins

### A) OPN blocking peptides

According to some embodiments, the OPN inhibitor is a blocking peptide. The OPN blocking peptide may be a peptide that blocks the interaction between OPN and its receptor. According to some embodiments, the OPN inhibitor comprises an amino acid sequence that is identical or similar to that portion of OPN that interacts with the OPN receptor but does not induce signal transduction via this receptor.

An "OPN blocking substance" may also be a peptide or protein comprising an amino acid sequence that (i) is identical or similar to part of the OPN receptor chain and (ii) interacts with OPN to thereby prevent OPN from binding to its receptor.

It is to be understood that a first amino acid sequence is similar to a second amino acid sequence if the first amino acid sequence is at least 70%, 80%, 90%, 95%, 97%, 98% or 99% identical to the second amino acid sequence. For example, the first amino acid sequence may differ from the second amino acid sequence in at most 1, 2, 3, 4, 5, 10 or more amino acids, e.g., by amino acid substitutions, deletions or additions.

The blocking peptide may be incorporated into a larger fusion protein to increase the stability of the protein and aid delivery to the target cell. The fusion protein can be designed to contain a specific protease cleavage site for recognition by a protease expressed in the target cell such that the peptide modulator is released from the fusion protein upon entry into the target cell. The blocking peptide may also be linked to a peptide that favours transport across the blood-brain barrier (BBB). For example, the peptide may be linked to a molecular Trojan horse (MTH) of ArmaGen Technologies. The MTH portion of the fusion protein triggers transport across the BBB via an endogenous receptor-mediated transport system. The blocking peptide may also be linked to another carrier, e.g., a dendrimer that favours transport across the BBB.

The blocking peptide can be synthesized using standard protein synthesis techniques as known in the art, for example, using chemical peptide ligation methods, including solid phase peptide synthesis, synthesize the peptide in the direction from the C-terminus to the N-terminus, including using an automatic peptide synthesizer. Alternatively, molecular biology techniques may be used to design an expression cassette that will encode a peptide modulator using standard molecular biology techniques known in the art. The expression cassette may be used in a suitable expression system. For example, the cassette may be contained in an expression plasmid and may be expressed in a bacterial or eukaryotic cell from which the peptide modulator can be isolated and purified. The expression cassette will contain an open reading frame encoding the blocking peptide, optionally as a complete peptide or as part of a chimeric or fusion peptide or protein from which the peptide can be released, for example by protease digestion. The expression cassette will also contain appropriate regulatory regions operably linked to the open reading frame, for example, a promoter region, which may be an inducible promoter region.

Alternatively, the blocking peptide may be included in a biomaterial that increases or induces cellular uptake of the blocking peptide or stabilizes the peptide upon systemic administration, for example by encapsulating the blocking peptide in a liposome formulation. The delivery of peptides and proteins into cells by liposomes is known and described in, for example, US Patent No. 6,372,720 and US 20030108597, incorporated herein by reference.

### Therapeutic administration and pharmaceutical compositions

Disclosed herein are methods of treating subjects suffering from cancer found to have an influx of myeloid cells: brain-resident microglia and peripheral macrophages with tumour-promoting activity, abbreviated GAMs (glioma-associated microglia and monocytes/macrophages). The method of treatment may include administering to a subject in need thereof a therapeutically effective amount of an OPN inhibitor to thereby reduce the protumorigenic activity of GAMs in the subject's tumour. According to some embodiments, the OPN inhibitor is administered locally, e.g., to a tumour, or systemically. The methods may maintain or stabilize tumour size, or reduce tumour size by at least 10%, 20%, 30%, 40%, 50%, 60%, 70%, 80%, 90% or more.

The term "treatment" refers to an approach to obtain beneficial or desired results, including clinical outcomes. Beneficial or desirable clinical results may include, but are not limited to, alleviating or ameliorating one or more symptoms or conditions, reducing the extent of the disease, stabilizing the disease, preventing the disease or condition from spreading or developing, delaying or slowing the progression of the disease, alleviation or improvement of the disease and remission (both partial and complete). "Treatment" can also mean prolonging a patient's survival beyond that expected in the absence of treatment or with existing treatment. "Treating" can also mean inhibiting the progression of the disease, temporarily slowing the progression of the disease, although more preferably it includes stopping the progression of the disease permanently.

The subject in need of treatment or prevention of disease may be a human. The subject may be a subject that has tumor, such as a subject that has cancer. Tumors that can be treated in accordance with the methods described herein include tumors that are infiltrated by macrophages with protumorigenic activity. The "protumorigenic" activity of macrophages refers to the ability of macrophages to support tumour development by, for example, participating in matrix remodelling, invasiveness, angiogenesis and suppressing the body's immune response, rather than initiating anti-tumour responses. Macrophages with protumorigenic activity are sometimes referred to as having the "M2 phenotype". Examples of tumours that are infiltrated by macrophages with protumorigenic activity are brain tumours, such as malignant gliomas. The glioma may be ependymoma, astrocytoma (e.g., glioblastoma multiforme), oligodendroglioma, or oligoastrocytoma. The glioma may be a low-grade or a high-grade glioma. A glioma may also be a supratentorial glioblastoma, an infratentorial glioblastoma, or a pontine glioma.

The peptides of the present invention can be administered to a patient using standard techniques known in the art. The therapeutic agent may be administered systemically or may be administered directly at the site where the target cell is located, e.g., in the brain. Site delivery includes topical administration, injection into a site (e.g., into the postoperative site), or surgical implantation, for example, in white adipose tissue or cellulose patches.

The concentration and amount of therapeutic agent to be administered will vary depending on the disorder being treated, the type of therapeutic agent that is administered, the mode of administration, and the age and health of the patient. However, one skilled in the art will be able to determine the appropriate amount.

To facilitate administration, the therapeutic agent may be formulated as a component of a pharmaceutical composition. Thus, in a further embodiment, a pharmaceutical composition is provided comprising a therapeutic agent and a pharmaceutically acceptable diluent. Therefore, pharmaceutical compositions for use in treating a disorder such as cancer are provided herein. The compositions may routinely include pharmaceutically acceptable concentrations of salts, buffering agents, preservatives, and various compatible carriers. For all delivery forms, the therapeutic agent may be formulated in physiological saline solution. The drugs may be incorporated into a liposome or other biomaterial useful for protecting and/or preserving the therapeutic agent until it is delivered to the target cell. The liposome may also help target the therapeutic agent to a desired location, e.g., a tumour.

The pharmaceutical composition may additionally comprise other therapeutic agents useful for treating the disorder, such as other agents for treating cancer. In some embodiments, one or more other agents are administered that block or reduce the protumoral activity of infiltrating macrophages. Such substances include hepatocyte growth factor (HGF) inhibitors; monocyte chemotactic protein (MCP1) inhibitors; MCP3 inhibitors and CXCRL1-CXCR1 inhibitors.

The pharmaceutical composition of the present invention may be prepared by known methods for preparing pharmaceutically acceptable compositions suitable for administration to patients, such that an effective amount of the therapeutic agent and any additional active ingredient or ingredients are combined in admixture with a pharmaceutically acceptable carrier. On this basis, pharmaceutical compositions include, but are not limited to, solutions of the therapeutic agent in association with one or more pharmaceutically acceptable carriers or diluents and are contained in buffer solutions at the appropriate pH and are isosmotic with physiological fluids.

The proportion and identity of the pharmaceutically acceptable diluent used with the drug depends on the chosen route of administration, compatibility with living cells, and standard pharmaceutical practice. Generally, the pharmaceutical composition will be formulated with ingredients that do not negate or impair the biological properties of the therapeutic agent.

The pharmaceutical composition of the present invention may be administered to a patient in a variety of forms, depending on the chosen route of administration, as will be understood by those skilled in the art. For example, the composition may be administered topically, surgically or by injection at the desired site. According to some embodiments, the therapeutic agent is administered topically or by injection (subcutaneously, intravenously, intramuscularly, etc.) directly to the desired site where the patient has target cells, e.g., white fat cells.

### Beneficial effects of the invention

Chemotherapy with the use of small molecule compounds, despite the lack of specificity for cancer cells and the development of drug resistance leading to the regrowth of drug-resistant cells, still remains the main method of cancer treatment. Small molecule compounds very effectively block the catalytic sites of enzymes and interactions between proteins, if there are "pockets" on the surface involved in the interaction that can bind the inhibitor. However, most proteins interact with their protein partners through vast and flat surfaces that can only be blocked by biological inhibitors such as peptides and proteins. Short peptides have a high affinity for the bound protein and have many advantages compared to protein-based medicinal substances. First of all, they are easy to synthesize, the peptide synthesis process is standardized and does not need to be optimized as in the case of protein production. In the synthesis, it is not necessary to be limited to L-amino acids only, D-amino acids can be used, as well as variously modified amino acids, various functional groups can be added. The use of non-natural amino acids makes it possible to increase resistance to degradation by various proteases. Peptides are safer to use, have reduced immunogenicity, and therefore are less likely to be toxic and cause unwanted side effects.

It should be emphasized that OPN is not expressed in the brain under physiological conditions. The peptides according to the invention, in addition to the advantages typical for peptide-based medicinal substances, have advantages resulting from the method of administration and the mechanism of its action. Peptides are administered into the brain. In a mouse model of glioblastoma, the peptide was injected directly into the brain using an osmotic pump that dosed the peptide at the same rate for 21 days. For patients with gliomas, the peptide will be administered intraoperatively, directly into the site created after surgical removal of the tumour in the form of cellulose wafers soaked with the peptide (currently, the so-called Gliadel-Carmustine wafers are administered). A special material soaked with the peptide will be placed in the postoperative site and left there. This method of administration bypasses the blood-brain barrier. The crossing of this barrier by drugs is a common problem in CNS therapies. When peptides are administered peripherally, they are rapidly degraded by numerous proteases present in the blood (intravenous administration) or in the digestive system (oral administration) or are cleared from the body by the liver and kidneys shortly after administration. There are not many proteases in the intercellular spaces of the brain, so the risk of the peptide being degraded is low. In addition, a similar peptide (G7), which is the subject of a patent application for its use in the treatment of glioblastoma (International Patent Application No. PCT/IL2019/051298), showed high stability in aqueous solution, in glioma cell supernatant and in serum. The G7 peptide was stable in aqueous solution for 3 weeks, which was important because the peptide administered to the mice was present in the osmotic pump during this time. This is also important information for optimizing the formulation of compounds, during which conditions are developed under which the compound (in this case, the peptide) will be stable for as long as possible. The G7 peptide was stable in the glioma cell supernatant for 14 days and was not degraded by proteases secreted into the media by the glioma cells. The half-life of the G7 peptide in mouse serum was 2 hours. This result is adequate in the context of the persistence of the peptide in the serum. The G7 peptide and the peptides of the present invention have the same length - 14 amino acids, which suggests that they may act similarly in terms of stability. Currently, the route of intravenous administration of the peptide is not considered, however, if necessary, there is always the possibility of modifying the peptide to increase resistance to proteolytic digestion, as well as encapsulation in the material, e.g., in liposomes.

The mechanism of action of the peptides of the present invention is also an advantage. Many peptides have low permeability through cell membranes, which poses a big problem in their use. The peptides according to the invention act extracellularly, they block the OPN protein-receptor interaction. The present protein is secreted by cells and therefore does not need to cross cell membranes. In addition, peptides that act intracellularly are more likely to be affected by proteases. Another advantage of the peptides of the present invention is that they block OPN cell signalling by binding the protein and not its receptor, such as, for example, peptides containing the RGD motif. Other cytokines also bind to the alphavbeta3 integrin receptor. Therefore, the use of a receptor-binding peptide (inhibitor) will also inhibit other signalling pathways, which in turn may cause undesirable side effects that are difficult to predict.

Due to the method of administration and the mechanism of action of the peptides of the present invention, the greatest disadvantages resulting from the use of peptides as medicinal substances have been avoided.

Sometimes peptides have difficulty penetrating deep into the tissue. This is a problem that can occur with the peptides of the present invention. However, this has not been observed so far. The peptide administered to mice with glioblastoma inhibited tumour growth, therefore there had to be adequate penetration of the peptide in the brain between the intercellular spaces. Biodistribution was tested for another peptide, peptide containing the RGD motif, which was designed in the laboratory of the present inventors (Ellert-Miklaszewska et al., 2016). A peptide with a FITC fluorescent tag was used, which was administered within an osmotic pump to mice with glioblastomas. The mice were then sacrificed and microscopic slides of the brain were prepared. The distribution of the peptide was analysed using a fluorescence microscope. The peptide spread throughout the parenchyma of the brain, although the highest signal was observed near the cannula that delivers the peptide to the tumour (unpublished data). On this basis, it can be assumed that the peptides of the present invention, of similar length, administered in the same way, will also have no problems with tissue penetration. In patients with gliomas, as mentioned in the above paragraph, the peptides of the present invention will be administered to the postoperative site. Most of the patients' glioblastoma cells will be surgically removed, so the peptide will not need to penetrate very deep into the parenchyma of the brain. Cancer cells that were not removed during surgery will be found around the postoperative site. However, if there are problems with biodistribution, it is possible to modify the peptide to increase it, for example by adding polyethylene glycol (PEG) molecules.

If the peptide of the present invention were to be used in the treatment of other cancers that occur outside the CNS, then there would be a need to administer the peptides peripherally. Probably, then, the peptide should be subjected to modifications aimed at increasing its stability, e.g., in the blood (depending on the route of administration) and half-life in the body. There are many ways to modify the peptide: from the use of unnatural amino acids, through the addition of various functional groups, cyclization of the peptide, which will reduce the susceptibility to proteolytic digestion and increase the persistence of the peptide. In order to increase the half-life in the body, for example, a peptide can be attached to albumin (many peptide molecules can be attached to one albumin molecule). Albumin has a high molecular weight (approximately 60 kDa) and has a half-life in the body of several days.

Currently, no solubility or biodistribution problems have been encountered with the peptides of the present invention. If there are limitations, there are a number of methods to overcome them as shown above.

### EXAMPLES

### Methods

### Demonstration of the role of human OPN protein in a glioblastoma model in vivo

Studies conducted by the present inventors indicate an important role of the OPN protein in the reprogramming of microglia and peripheral macrophages by glioblastoma cells to a protumorigenic phenotype (Ellert-Miklaszewska et al. 2016). In a rat model of glioblastoma, the key role of OPN in the modulation of the antitumour response has been demonstrated. Investigating the contribution of human OPN to human glioblastoma growth is important in the context of using a peptide that binds human OPN produced by glioma and can block communication between the glioma and the microenvironment. The U87 MG RFP human glioblastoma cell line was established with silenced expression of the OPN protein (shOPN). It was shown that although silencing the OPN protein did not change the proliferation of these cells, the cells had impaired interaction with microglia and decreased invasiveness in the presence of microglia. The number of glioma cells actively penetrating the Matrigel Matrix layer (BD Biosciences, extracellular matrix surrogate) in the presence of SV40 human microglial cells was counted. Microglial cells are plated on 24-well plate, after 24 hours, inserts previously covered with Matrigel suspension are placed in the wells, and glioblastoma cells are seeded on its surface. The tested compounds, potentially blocking the invasiveness of cells, are added in a certain concentration to the medium in both the lower and upper layers of the system. After 18 hours of incubation, the Matrigel is removed and the cells on the insert membrane (invading cells) are fixed and stained with a DAPI fluorescent marker. Slides were then photographed using a fluorescence microscope and cells were counted using Image J software (imagej.nih.gov/ij/). The invasiveness of shOPN glioma cells in the presence of SV40 was shown to be lower compared to control cells (transfected with neutral shNeg) (Figure 1A). These results indicate that the human OPN protein is crucial for communication between glioma cells and microglia and microglia-dependent invasiveness.

Subsequently, shOPN and shNeg human glioma U87 MG RFP cells were implanted into Athymic Nude (Foxn1nu) mice and tumour sizes were assessed after 21 days based on histological analyses. Fluorescent protein RFP (Red Fluorescent Protein) produced by U87 MG RFP cells and a fluorescence microscope were used to visualize tumours on slides. Mice implanted with shOPN cells did not develop tumours or the tumours were small compared to control animals implanted with U87 MG RFP or shNeg cells (Figure 1B).

### Design, identification and selection of OPN interacting peptides

The first step in identifying OPN-binding peptides with potential therapeutic use was the design and creation of a peptide library. OPN proteins are secreted extracellularly and bind to integrin receptors consisting of alphavbeta3 and alphavbeta5 subunits located on the cell surface. On the basis of the crystallographic structures of the complexes of the studied proteins and their receptors, fragments of receptors potentially involved in the binding of these proteins were identified. 14-amino acid peptides (designated as I) were designed covering fragments of the receptor sequences relevant for interaction with the ligand, including 26 peptides derived from the alphav subunit and 26 peptides from the beta3 subunit of the integrin receptor. The peptides are designed in the form of overlapping fragments so that each subsequent peptide coincides with the last 7 amino acids of the previous peptide.

In order to identify OPN binding peptides, special glass microarrays were used, which included a designed library of synthesized peptides (JPT Peptide Technologies GmbH). These microarrays were incubated with recombinant human OPN protein (rhOPN), unbound proteins were removed by washing several times, and then the microarrays were blocked with a 5% skimmed milk solution. Proteins that bound to the peptides were detected using a primary antibody directed against the Myc tag (the C-terminus of a rhOPN protein had a Myc tag) and a secondary antibody coupled to the Dylight800 fluorescent tag, which enabled the detection of interactions and the quantification of the signal from bound protein. OPN bound the following peptides: I1, I2, I10, I36, I41, I47 (Figure 2A).

To verify the obtained results, the ELISA method assessing peptide-protein binding was used. In the assay, 96-well ELISA plates were coated with recombinant OPN protein. Uncoated wells were used as controls. Unbound proteins were then washed away and the remaining non-specific binding was blocked with bovine serum protein (BSA). Test peptides with N-terminal biotin attached were added. After incubation, unbound peptides were removed by washing several times. Peptides that bound to OPN were detected using a primary anti-biotin antibody and a secondary antibody linked to horseradish peroxidase. This enzyme catalyses a reaction in which a coloured product is formed from the added substrate. The amount of bound peptides was proportional to the amount of coloured product, which was measured using a spectrophotometer. Of the 52 integrin receptor-derived peptides tested, 9 OPN-binding peptides were identified: 13, I4, I20, I32, 138, I40, I47, I49, I50 (Figure 2B). The following peptides were selected for further analysis: **I1, I2, I4, I20, 132, 138, I40, I47, I49, I50.**

### Evaluation of potential cytotoxicity of peptides

To rule out the potential toxicity of OPN-binding peptides, the effect of the peptides on microglial cell viability and U87 MG human glioma was tested. The MTT metabolism test was used, in which the amount of reduced MTT (by mitochondrial enzymes) is directly proportional to the number of living, metabolically active cells and measured with a spectrophotometer. Mouse BV2 microglia cells and U87 MG human glioma cells were treated with the tested peptides at a concentration of 100 µM for 18 hours. Of the 14 OPN-binding peptides tested (I1, I2, I3, I4, I10, I13, I20, I26, I32, 138, I40, I47, I49 and I50), only I38 and I47 reduced mouse microglial cell viability by 50% compared to the viability of untreated cells (Figure 3). **These peptides were excluded from further studies.**

### Example 1. Identification of peptides blocking microglia-dependent glioma cell invasiveness

The effect of selected OPN-binding peptides on the invasiveness of U87 MG cells stimulated by the presence of microglial cells was investigated. Peptides were added to the co-culture of U87 MG and BV2 cells at a concentration of 100 µM for 18 h. Of the 12 OPN-binding peptides tested (I1, I2, 13, I4, I10, I13, I20, 126, I32, I40, I49 and I50), peptides: **I40, 149, I50** inhibited microglia-dependent glioma cell invasiveness; the control was basal cell invasion in the presence of microglia defined as 100%. The number of invasive cells was reduced by up to 75% compared to control (Figure 4). For additional verification of the activity of the peptides in another experimental model, I40, I49, I50 peptides were tested using Matrigel in a system containing U87 MG cells and SV40 human microglia cells. Control peptides (*scrambled*) were also included in these analyses. The I50S peptide was not used because it did not dissolve in the solvent, i.e. 100% DMSO (dimethylsulfoxide), which was used to dissolve all the peptides. Therefore, the effect of DMSO at the corresponding concentration was tested in all experiments.
Peptides: **I40, I49 and150** inhibited the invasiveness of U87 cells in the presence of human SV40 microglia, control peptides: I40S and I49S had no effect on invasiveness (Figure 4).

### Example 2. Investigation of the anticancer activity of the peptide in a mouse model of human glioblastoma

The anticancer effect of the I49 peptide has been tested. The model of human glioblastoma U87 MG RFP cells implanted in the brain of nude (immunodeficient) mice was used. Peptides were administered via osmotic pumps delivering the peptide directly to the tumour at a constant rate. The peptide is well soluble in water and a concentration of 100 µM was obtained without any problems. Peptide I49 or control peptide I49S were administered using pumps for 3 weeks, pumps were installed in the mouse brain immediately after implantation of U87 MG RFP cells. Magnetic resonance imaging (MRI T2) was used to assess the size of the tumours. A reduction in tumour growth was observed in mice that received the I49 peptide pump compared to the control group (Figure 5).

It is to be understood that the phraseology or terminology used herein is for the purpose of describing the embodiments, and that the means, materials, and steps for carrying out the various experiments disclosed may take various alternative forms without departing from the invention.

### Application of the invention

The inventors of the present invention identified new molecular targets that enabled the development of a novel targeted strategy for the treatment of gliomas. The new target is the OPN protein secreted by glioblastoma cells and not expressed under physiological conditions (except during embryonic development). Osteopontin regulates the accumulation and programming of microglial and macrophage cell functions by acting through integrin receptors on the surface of these cells. An inhibitor, short peptides of the present invention interfering with the binding of OPN to its receptor, was invented and demonstrated to be effective in an animal model of human glioblastoma. The use of the present invention requires refinement of elements such as the route of administration and dosage, the appropriate formulation of the drug candidate as well as the ADME/tox profile. If the set parameters are achieved, the effect of the drug candidate will be verified in clinical trials, which will be aimed at introducing a new active substance to the pharmaceutical market. The use of short interfering peptides is a new strategy, but gaining interest from pharmaceutical companies that conduct clinical trials on short interfering peptides (e.g., cilengitide by Merck) in cancer therapy (MacDonald et al., 2013; Mullins et al., 2013). The obtained peptides will be administered into the brain, to the site formed after the removal of the tumour. This approach will ensure that they act locally, in the place where cancer cells were present. Therefore, it is expected that the side effects of the future drug will be almost completely eliminated in patients, as well as the barriers to the delivery of the active substance to its place of action, such as the blood-brain barrier, will be bypassed. Intratumoural administration of the drug will also lower the barrier to the rapid application of a new drug based on the invention, as it will be easy to incorporate the administration of this drug into currently used therapeutic pathways in the context of glioblastoma. The most important, however, in this context seems to be the therapeutic potential resulting from the inhibition of OPN function.

## Claims

1. A synthetic peptide for use in inhibiting OPN activity, wherein said peptide comprises an amino acid sequence selected from the group consisting of: the amino acid sequence set forth in SEQ ID NO: 1, the amino acid sequence set forth in SEQ ID NO: 2; and the amino acid sequence set forth in SEQ ID NO: 3, or variants thereof having 70%, 80%, 90%, 95%, 97%, 98% or 99% sequence identity.

2. The synthetic peptide according to claim 1, wherein said peptide is 14 amino acids in length.

3. The synthetic peptide according to claim 1 or 2, wherein said peptide is a cyclic peptide.

4. A pharmaceutical composition comprising a synthetic peptide according to any one of claims 1 to 3 and a pharmaceutically acceptable carrier.

5. The pharmaceutical composition according to claim 4, wherein said pharmaceutically acceptable carrier is selected from the group consisting of: an aqueous solution, vegetable oil, alcohol, polyethylene glycol, propylene glycol or glycerine.

6. The synthetic peptide according to any one of claims 1-3 or a pharmaceutical composition according to claims 4-5 for use in the treatment of glioma.

7. The synthetic peptide or pharmaceutical composition for use according to claim 6, wherein said glioma is selected from the group consisting of: ependymoma, astrocytoma, oligodendroglioma, glioblastoma, or mixed glioblastoma.

8. A kit for use in the treatment of glioma, said kit comprising the pharmaceutical composition according to one or more of claims 4 to 5 and instructions for use of said kit.

## Patentansprüche

1. Ein synthetisches Peptid zur Hemmung der OPN-Aktivität, wobei das erwähnte Peptid eine Aminosäuresequenz umfasst, die aus einer Gruppe ausgewählt ist, die umfasst: eine Aminosäuresequenz, die in SEQ ID NO: 1, eine Aminosäuresequenz, die in SEQ ID NO: 2; und eine Aminosäuresequenz, die in SEQ ID NO: 3 festgelegt ist, oder Varianten davon mit 70 %, 80 %, 90 %, 95 %, 97 %, 98 % oder 99 % Identität der Sequenz.

2. Das synthetische Peptid nach Anspruch 1, wobei das Peptid eine Länge von 14 Aminosäuren aufweist.

3. Das synthetische Peptid nach Anspruch 1 oder 2, wobei das Peptid ein zyklisches Peptid ist.

4. Eine pharmazeutische Zubereitung, die ein synthetisches Peptid nach einem der Ansprüche 1 bis 3 und einen pharmazeutisch zulässigen Trägerstoff enthält.

5. Die pharmazeutische Zubereitung nach Anspruch 4, wobei der pharmazeutisch zulässige Trägerstoff aus einer Gruppe gewählt wird, die eine wässrigen Lösung, Pflanzenöl, Alkohol, Polyethylenglykol, Propylenglykol oder Glycerin umfasst.

6. Das synthetische Peptid nach einem der Ansprüche 1 - 3 oder eine pharmazeutische Zubereitung nach Anspruch 4 - 5 zur Verwendung bei der Behandlung von Gliomen.

7. Das synthetische Peptid oder eine pharmazeutische Zubereitung zur Verwendung nach Anspruch 6, wobei das Gliom aus einer Gruppe ausgewählt ist, die umfasst: Ependymom, Astrozytom, Oligodendrogliom, Glioblastom oder gemischtes Glioblastom.

8. Eine Garnitur zur Verwendung bei der Behandlung von Gliomen, wobei die Garnitur die pharmazeutische Zubereitung nach einem oder mehreren Ansprüchen 4 bis 5 und eine Gebrauchsanweisung für die erwähnte Garnitur enthält.

## Revendications

1. Peptide synthétique pour son utilisation dans l'inhibition de l'activité des OPN, ledit peptide comprenant une séquence d'acides aminés sélectionnée dans le groupe constituée par: la séquence d'acides aminés définie dans la SEQ ID N°: 1, la séquence d'acides aminés définie dans la SEQ ID N° : 2, et la séquence d'acides aminés définie dans la SEQ ID N° : 3, ou des variantes de celles-ci ayant une identité de séquence de 70 %, 80 %, 90 %, 95 %, 97 %, 98 % ou 99 %.

2. Peptide synthétique selon la revendication 1, dans lequel peptide ayant une longueur de 14 acides aminés.

3. Peptide synthétique selon la revendication 1 ou 2, dans lequel ledit peptide est un peptide cyclique.

4. Composition pharmaceutique comprenant un peptide synthétique selon l'une quelconque des revendications 1 à 3, et un support pharmaceutiquement acceptable.

5. Composition pharmaceutique selon la revendication 4, dans laquelle le support pharmaceutiquement acceptable est choisie dans le groupe constitué par"une solution aqueuse, une huile végétale, de l'alcool, du polyéthylène glycol, du propylène glycol ou de la glycérine.

6. Peptide synthétique selon l'une quelconque des revendications 1 à 3 ou une composition pharmaceutique selon les revendications 4 à 5 pour son utilisation dans le traitementdu gliome.

7. Peptide synthétique ou la composition pharmaceutique à utiliser selon la revendication 6, dans lequel ledit gliome est choisi parmi le groupe constitué de l'épendymome, astrocytome, oligodendrogliome, glioblastome ou glioblastome mixte.

8. Kit destiné au traitement du gliome, ledit kit comprenant la composition pharmaceutique selon une ou plusieurs des revendications 4 à 5 et les instructions d'utilisation dudit kit.
